# EUROPEAN PATENT APPLICATION

(11) **EP 1 671 581 A1**
(43) Date of publication of application: **21.06.2006**
(21) Application number: 05027067.7
(22) Date of filing: 12.12.2005
(51) Int. Cl.: A61B 5/0285

(54) **Method and device for detection of arterial occlusions in arms and legs**

(30) Priority: 14.12.2004 LV 040144
(71) Applicant: Latvijas Universitate, Riga 1098 (LV)
(72) Inventor: Spigulis, Janis, Riga 1046 (LV); Erts, Renars, Lielvarde Ogres rajons 5070 (LV); Ozols, Maris, Jelgava 3000 (LV); Kukulis, Indulis, Riga 1024 (LV); Lihacovs, Aleksejs, Riga 1002 (LV)
(74) Representative: Fortuna, Aleksandra

(57) **Abstract**

The invention relates to angiology, in particular - to determination of magistral arterial occlusions in arms and legs by means of non-invasive, e.g. photoplethysmographic (PPG) multi-channel registration of blood pulsation signals from anatomically symmetrical sites of arms and/or legs with subsequent signal processing and assessment of difference(s) of the pulse wave transit time accordingly to the measured time shift(s) between the corresponding pulsating signals. The proposed device for detection of the arterial occlusions in arms and/or legs comprises optical PPG contact probes (1, 2, 3, 4) in amount that corresponds to the number of used channels, electronic amplifiers (5, 6, 7, 8) that are amplifying the output signals of all contact probes, microprocessor (9) that provides transfer of the above mentioned signals to the processing units (10 - for arms, 11 - for legs), and the output unit (12) - panel, monitor or similar device that provides indication or signalling about occlusion at the respective arm or leg, e.g. by switching on the corresponding light-emitting diode (12.1, 12.2, 12.3, 12.4).

## Description

The invention relates to angiology, in particular - to determination of magistral arterial occlusions in arms and legs.

Effective existence and functioning of any organ requires sufficient blood supply. Control of blood circulation parameters is important both in cases of cardiovascular circulatory disturbances, and in prophylaxis. Arterial narrowings (occlusions) in arms and legs that hinder or even block blood supply constitute one of the most perilous diseases of blood circulation disturbances and may cause serious organic changes.

Several methods of diagnostics of arterial occlusions are known, e.g. arterial oscillography, local registration of blood pressure and determination of the ankle/arm index.

The method of arterial oscillography is used to register pulsatile vibrations of arterial walls. The arterial oscillogram is recorded by means of a cuff placed on the limb region, where the pressure changes gradually. The oscillogram is taken in decompression - decreasing the pressure in the oscillometric cuff. For detection of an occlusion the subject has the output level of oscillations in his foot or lower shin recorded. The maximum oscillation value is measured in millimetres and typically designated as the oscillometric index. Variations within the average range from 4 to 30 mm are considered as normal. The extent of oscillometric index decrease conforms to the gravity of the disease only approximately. In terms of diagnostics, it is more important to compare the value of such index at the respective levels of symmetrical extremities. Functional tests, including occlusion tests, are performed in cases where analysis of oscillation amplitude in rest precludes from making reliable diagnostic conclusions,. After the output level of oscillations in the lower shin is recorded, an arterial constrictor is applied on the distal part of the thigh, where a compression chamber increases the pressure up to 220 - 240 mmHg. In five minutes, the constriction for blood vessel strangulation is removed. In 10 - 30 seconds after decompression of the thigh, an oscillogram is taken. After short-time strangulation of their leg arteries, the oscillation amplitude of subjects with organic arterial damages in legs decreases, and that of healthy subjects increases (J. Ska̅rds, I. 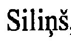 , "Loceklu asinsvadu slimi̅bas", Riga, Zvaigzne, 1967, pp. 45-57; A. Valtneris, "Asinsrites Fizioloǵija", Riga, Zvaigzne, 1979, pp. 102-112).

The method of local registration of blood pressure is used to measure the blood pressure in legs at three levels - in the lower thigh, in the upper shin and in the lower shin. Normally the difference in the blood pressure in both legs does not exceed 5-8 mmHg. To detect the arterial occlusions in arms, the local blood pressure is registered in the middle arm and in the upper and lower forearm.

For detection of an occlusion the examined subject has the values of the systolic blood pressure measured at different levels of his extremities. The results are assessed by comparing the pressure differences between similar levels of symmetrical extremities (the horizontal pressure gradient) and between different levels of the same extremity (the vertical pressure gradient). Pressure differences exceeding 10 - 20 mmHg indicate arterial stenosis, and those exceeding 30 - 40 mmHg indicate arterial occlusion. If required, an occlusion test may supplement such examination. After the output level of the blood pressure is determined in the lower shin, blood circulation is stopped at the level for five minutes. When the local blood pressure is registered in the shin again in 10 - 30 seconds after the circulation resumes, it is established that the blood pressure in the bad leg falls fast (by more that 10 - 15 mmHg) for subjects with the occlusion in legs. In similar circumstances, the blood pressure of healthy subjects does not change or decreases by less than 5 - 10 mmHg (Segmental limb systolic measurement, *International Angiology,* Vol. 19, Suppl. 1 to No.1, p. 71, 2000).

The ankle/brachial index (ABI) is the ratio of the blood pressure in the artery of the foot (lower shin) to that of the brachial artery. It is impossible to determine the site of the arterial narrowing by means of the ABI, however, the method is precise and convenient to use for diagnostics of atherosclerosis of lower extremities, as well as to assess the gravity of disease (A. La̅cis, *"Perife̅risko arte̅riju okluzi̅vo slimi̅bu diagnostika un* 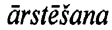 ", Riga, Nacionälais apgäds, 2004, pp. 35-38). There is a specific device (see e.g. Superior-and-inferior-limb blood-pressure index measuring apparatus, EP 1 240 866, A61B5/022, 2002) for determining the ABI based upon measurement of the blood pressure in the arm and the ankle regions. It is possible to determine the degree of arterial stenosis by means of this device.

Disadvantages of the known methods cover inconvenience of their use (constrictors, pressure cuffs with compression and decompression equipment, etc.), complicated procedures (several manipulations in a specific order), relatively long duration (typically about 30 minutes) and difficulties to apply the method outside hospitals (e.g. in field or domestic conditions), because of the massive stationary equipment.

The method, as described in the article "Segmental limb systolic measurement", *International Angiology,* Vol. 19, Suppl. 1 to No. 1, p. 71, 2000, has been chosen as a prototype of the invention.

The invention seeks to ensure more convenient, simpler and faster detection of arterial occlusions in arms and legs, available also outside hospitals.

To achieve the above goal, non-invasive multi-channel registration of blood pulsations in proximity of the arteries of extremities is used with sufficient time resolution, e.g. with application of the photoplethysmographic (PPG) method to determine changes in the blood volume. Several applications of the photoplethysmographic method are known for control of cardiovascular parameters: for registration of the frequency of heart activity (Apparatus and method for detecting heartbeat using PPG, EP 1 354 553, A61B5/024, 2003), for determining the temporal parameters, peripheral resistance and the mean pressure of the arterial system (Apparatus and method for measuring the variability of cardiovascular parameters, US 5 862 805, A61B19/00, 1999), for non-invasive determining of the blood pressure without using pressure cuffs (Method and apparatus for non-invasove, cuffless, continuous blood pressure determination, US 5 865 755, A61B5/00, 1999), for non-invasive determining of the diastolic blood pressure (Method for systolic blood pressure measurement, US 6 402 696, A61B5/02, 2002), etc.

As the heart muscles contract, a certain blood quantity is thrown out, the aortal pressure increases for a short time, and a pulse wave transits along the arteries with a specific velocity and causes regular vascular volume pulsations. The vascular anatomy is symmetrical, so the pulse wave signals take the same time to reach bilaterally symmetrical sites of the extremities of humans without pathological changes in blood vessels (e.g. the index fingers of the right and the left hands, or the little toes of the right and the left feet). In cases where pulsations in tissue blood volume are registered in such sites, healthy subjects have both pulsation signals registered simultaneously or synchronously. Unilateral arterial occlusion causes additional vascular resistance, which results in increased pulse wave transit time, hence in pathological cases some time shift may be expected between the two pulsation signals.

Several methods of non-invasive registration of blood pulsations are known, including the optical method. In cases where tissues are subject to optical radiation, the absorption of the radiation that propagates through tissues changes synchronously with the changing blood volume. By using a photoplethysmographic (PPG) contact probe that incorporates a continuous optical radiation source and a photo-detector, the time dependence of intensity for the tissue-transmitted or back-scattered radiation may be recorded, which gives adequate description of the tissue blood volume pulsations in real time. In the absorption PPG method, the emitter and the photo detector are placed on opposite sides of the body parts (finger, ear, etc.) and the resulting changes in the tissue-transmitted radiation are registered, while in the reflection (remission) PPG method, both elements contact the skin surface near each other and the time dependence of the tissue back-scattered radiation is registered (J. Spigulis, R. Erts, I. Kukulis, M. Ozols, K. Prieditis, Optical multi-channel sensing of skin blood pulsations, *Proc. SPIE,* Vol. 5459, pp. 46-53, 2004).

The invention will now be described by way of example and with reference to the accompanying drawings in which:
Figure 1 illustrates the correlation between local blood pressure differences of right - left extremities dP and the time shifts of the respective PPG signals Δt for patients with arterial occlusions;
Figure 2 explains determination of Δt pursuant to the time shift of the minima points of two PPG signals (PPG signals of the right and left leg are shown by means of a continuous and a dash line accordingly; there is an occlusion in the left leg); and
Figure 3 presents the block diagram of a four-channel device for simultaneous detection of arterial occlusions in arms and legs.

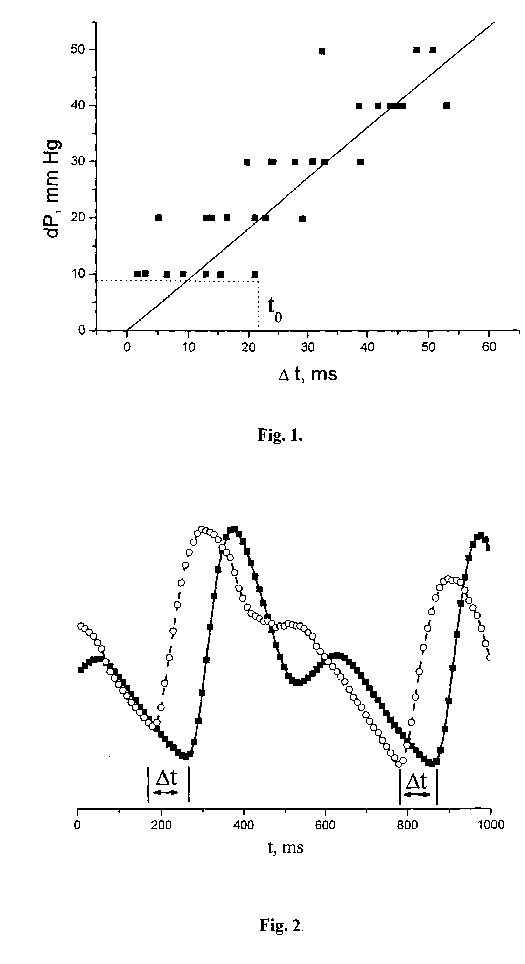

A clinical study was performed on 50 patients with diagnosed arterial occlusions in legs or arms, and a correlation (Fig. 1) was obtained for the blood pressure difference between right and left extremities dP measured by means of the local blood pressure method, and a respective time shift Δt between the bilateral PPG signals (J. Spigulis, R. Erts, I. Kukulis, M. Ozols, K. Prieditis, Optical multi-channel sensing of skin blood pulsations, *Proc. SPIE,* Vol. 5459, pp. 46-53, 2004). All measurements were taken in horizontal position of the body after 5-minute relaxation at the room temperature of 23 ± 2°C. Pursuant to the correlation obtained, it is possible to assess the threshold value to of the time shift Δt between the PPG signals; all Δt values exceeding the threshold then indicate existence of the arterial occlusion.

By means of multi-channel PPG registration of signals with subsequent signal processing, blood pulsation signals are taken from each contact site simultaneously. The time of the pulse wave arrival for the left-hand finger is marked as t₁, that for the righthand finger as t₂, left-foot toe - t₃, and right-foot toe - t₄. The time difference between the pulse wave arrivals (that may be determined, for example, by shift of the PPG signal minima points - Fig. 2) for arms is Δt_{R} = t₁ - t₂, and for legs Δt_{K} = t₃ - t₄. The values differ from zero in the following four cases: Δt_{K} > 0, Δt_{K} < 0, Δt_{R} > 0, Δt_{R} < 0. For instance, in cases where |Δt_{K,}| > t₀, where t₀ is the threshold value of arterial occlusion of the leg, it should be concluded that one of the legs has an arterial occlusion - with Δt_{K} > 0 it is occlusion of the right leg, and with Δt_{K} < 0 it is occlusion of the left leg. Similarly, where the respective limit value of occlusion of the arm is exceeded, with Δt_{R} > 0 it is identified as occlusion of the right arm, and with Δt_{R} < 0 as occlusion of the left arm.

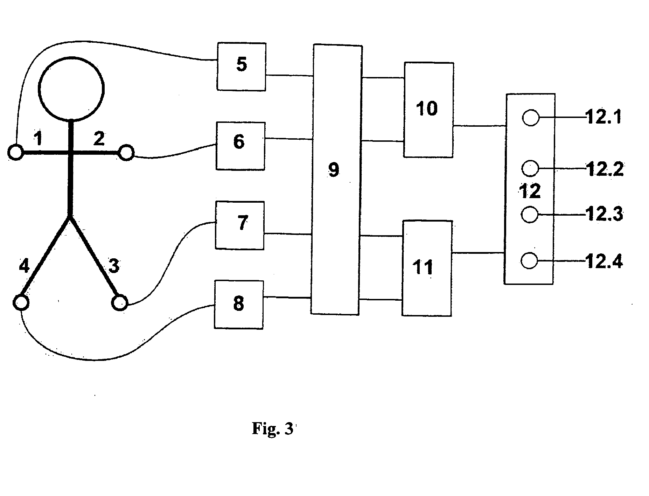

The process of detection of occlusions is described below:
- optical PPG contact probes are connected to anatomically symmetrical sites of the patient's extremities (e.g. to the little toes of both feet or to the index fingers of both hands);
- PPG signals are registered over the period exceeding five pulse beats (e.g. 10 sec.);
- the signals received are processed by means of the software, and the mean value of the time shift Δt between both signals is determined;
- the obtained mean value Δt is compared with the threshold value of occlusion to to be determined, for example, from the correlation diagram (Fig. 1);
- In cases where the threshold value is exceeded, the conclusion is made on the occlusion in the respective arm or leg pursuant to whether Δt is positive or negative;
- to obtain additional information on the specific occlusion, the mean value Δt according to the degree of gravity of the occlusion is assessed, the shape parameters of the PPG signals registered in all channels are compared and changes (fluctuations) of the time shift Δt during the measurements are recorded (J. Spigulis, R. Erts, I. Kukulis, M. Ozols, K. Prieditis, Optical multi-channel sensing of skin blood pulsations, *Proc. SPIE,* Vol. 5459, pp. 46-53, 2004).
   The proposed device (Fig. 3) for detection of the arterial occlusions in arms and/or legs comprises optical PPG contact probes 1, 2, 3, 4 in amount that corresponds to the number of used channels (generally, 2n, where n = 1, 2, 3, ...; a 4-channel version is considered herein), electronic amplifiers 5, 6, 7, 8 that are amplifying the output signals of all PPG contact probes, processor 9 that provides transfer of the above mentioned signals to the processing units: 10 - for arms, 11 - for legs with software for analysis of the shift, and the output unit 12 - panel, monitor or similar device that provides indication (signalling) of the detected occlusion and/or display of the value of time shift measured accordingly 12.1, 12.2, 12.3, 12.4, and/or shows the time shift fluctuations (or parameters of such fluctuations), and/or compares the shapes (or shape parameters ) of the PPG signals registered in different channels.
   The device makes it possible to perform a complex non-invasive arterial examination : qualitative detection of arterial occlusions (stating the specific arm or leg), quantitative description of occlusions (according to the measured value Δt, the nature of Δt fluctuations, and comparison of shape parameters of the PPG signals registered in different channels) and specification of the occlusion localisation (stating the specific part of the leg or arm).
   Occlusion in the extremity is established in cases where the mean time shift between the respective PPG signals registered in anatomically symmetrical sites of both legs (arms) exceeds the threshold value to. As a result, a light emitting diode corresponding to the respective arm or leg switches on in the output unit of the device, or a different way of indication/signalling is used.
   According to the correlation between the values of time shift of the registered PPG signals and the data obtained by other means of detecting occlusions (e.g. the difference between measured local blood pressures in anatomically symmetrical sites of arms/legs - Fig. 1), it is possible to assess the gravity or the degree of peril of the occlusion. The output unit of the device displays the mean numerical value of the respective time shift in digital form together with the quality indication (e.g. switching on the light emitting diode corresponding to the respective arm or leg). The nature of Δt fluctuations during the measurements and comparison of shape parameters of the PPG signals registered in different channels provides additional diagnostic information. This helps the physician to assess the degree of peril of the occlusion and the strategies of further treatment.
   It is possible to specify localisation of the occlusion by means of registration of 2n - channels (n = 2, 3, 4, ...),- for example, by establishing whether it is in the thigh or the shin. After an occlusion is detected in any extremity, the pair of the extremities is divided into several symmetrical ranges and the PPG contact probes are connected. Then the respective time shift Δt > t₀ is obtained (or not) for each pair of symmetrical registration sites and shown in the output unit of the device. The occlusion localisation is determined in the direction from the heart towards the tips of fingers or toes of extremities. For example, in cases where the signals of both thighs have no time shift, but those of shins do, the occlusion is located in the femoral artery of the respective leg.
   The examples which follow explain the invention:

### Example 1

The patient is a man of 65 years with arterial occlusion in the left arm (diagnosed by means of the method of local blood pressure: the blood pressure in the right arm is 135 mmHg, and that in the left arm is 115 mmHg). The patient is laid horizontally and the PPG probes are connected to the index fingers of the right and the left hands and the big toes of the right and the left feet. Over a minute, the PPG signals are registered in all the four channels. As result, the signal from the left arm is stated to arrive later than that from the right arm by 0.024 second on the average, which exceeds the limit value of occlusion; no time shift is detected between the PPG signals from the right and the left legs.

### Example 2

The patient is a woman of 59 years with arterial occlusion in the right leg (diagnosed by means of the method of local blood pressure: the blood pressure in the right leg is 130 mmHg, and that in the left leg is 90 mmHg). The patient is laid horizontally and the PPG probes are connected to the index fingers of the right and the left arms and the middle toes of the right and the left feet. Over a minute, the PPG signals are registered in the four channels simultaneously. As result, the signal from the right leg is stated to arrive later than that from the left leg by 0.052 second on the average, which exceeds considerably the limit value of occlusion; no time shift is detected between the PPG signals from the right and the left arms.

The proposed method and device make detection of arterial occlusions more convenient - PPG contact probes for fingers are much handier than pressure cuffs that are used in the known methods. It is simpler because no additional manipulations are required during the measurements (e.g. changing the controlled pressure in the cuffs on arms or legs). The minimum time required to detect occlusion is less than one minute, which is substantially faster against the prototype method (about 30 minutes). The proposed method may be used for diagnostics both in hospitals and in field or domestic conditions, because the device for application of this method is lightweight (< 5 kg) and may be battery-operated. The method is non-invasive and may be applied simultaneously with other methods for examination of the body physiological condition, it is applicable outside hospitals, e.g. for early screening.

## Claims

1. A method for detection of arterial occlusions in arms and/or legs by means of comparison of physiological parameters of the blood circulation system, **characterized in that** for more convenient, simpler and faster detection of occlusions in arms and legs, non-invasive, e.g. photoplethysmographic (PPG), multi-channel registration of blood pulsation signals from anatomically symmetrical sites of arms and/or legs is used with subsequent signal processing and assessment of difference(s) of the pulse wave transit time accordingly to the measured time shift(s) between the corresponding pulsation signals.

2. The method according to claim 1, **characterized in that** the time shift between the PPG signals with the specific threshold value of occlusion is compared for qualitative detection of the occlusions.

3. The method according to claims 1 and 2, **characterized in that** the threshold value is determined according to correlation of the difference between measured local blood pressures and the time shift between the PPG signals.

4. The method according to claims 1-3, **characterized in that** two-channel PPG registration from symmetrical fingertips of both arms is used for detection of arterial occlusions in arms.

5. The method according to claims 1-3, **characterized in that** two-channel PPG registration from symmetrical tips of toes of both feet is used for detection of arterial occlusions in legs.

6. The method according to claims 1 - 3, **characterized in that** four-channel PPG registration from symmetrical tips of fingers of both arms and toes of both feet is used for simultaneous detection of arterial occlusions in arms and legs.

7. The method according to claims 1-6, **characterized in that** 2n-channel (n = 1, 2, 3, ...) PPG signal registration from any symmetrical sites of arms and/or legs is used for specification of the occlusion localisation.

8. The method according to claim 7, **characterized in that** the occlusion localisation is specified by determining and mutually comparing time shifts between PPG signals for each pair of symmetrical registration sites.

9. The method according to claims 1 - 8, **characterized in that** reflection and/or absorption PPG method is applied for detection of occlusions.

10. The method according to claims 1 - 9, **characterized in that** numerical values of time shift of the corresponding PPG signals are used for qualitative and/or quantitative assessment of occlusions.

11. The method according to claim 10, **characterized in that** the time shift is determined according to the moments of time of corresponding minima and/or maxima of both PPG signals and/or points selected on the systolic front, by subtracting them from each other accordingly.

12. The method according to claims 1-11, **characterized in that** additionally parameters of changes (fluctuations) of momentary values of the PPG signal time shifts and/or comparison of shapes (shape parameters) of the PPG signals registered in different channels are used for diagnostics of occlusions.

13. The method according to claims 1 - 12, **characterized in that** blood pulsation signals registered otherwise than PPG signals are used for assessment.

14. A device proposed for detection of the arterial occlusions in arms and/or legs, comprising optical PPG contact probes in amount that corresponds to the number of used channels, an electronic amplifier that is amplifying the output signals of all contact probes, a processor that provides transfer of the above mentioned signals to the processing unit and the output unit, e.g. a panel, a monitor or a similar device that provides indication or signalling of the detected occlusion.

15. The device according to claim 14, **characterized in that** special software stored in a data medium is used for implementation of the method pursuant to claims 1 - 13 above.

16. The device according to claims 14 and 15, **characterized in that** an output unit provides qualitative indication (signalling) of the detected occlusion, e.g. switching on a light-emitting diode or a different emitter corresponding to the respective arm or leg.

17. The device according to claims 14 - 16, **characterized in that** an output unit provides quantitative indication of the detected occlusion, e.g. by using digital displays according to the number of channels, each showing the mean value of time shift between the corresponding PPG signals.

18. The device according to claim 17, **characterized in that** additional signalling about occlusion detected in an arm and/or leg (e.g. by a flashing light signal) is provided in cases where the threshold value is exceeded.

19. The device according to claims 14 - 18, **characterized in that** an output unit provides information on changes (fluctuations) of momentary values of the PPG signal time shift and/or on shapes of the PPG signals registered in different channels and/or corresponding shape parameters.
